Europäisches Patentamt

(19) European Patent Office

Office européen des brevets

(11) Publication number: **0 328 370**
**A2**

(12) **EUROPEAN PATENT APPLICATION**

(21) Application number: **89301212.0**

(22) Date of filing: **08.02.89**

(51) Int. Cl.⁴: **A 61 L 9/03**

(30) Priority: **08.02.88 GB 8802772**

(43) Date of publication of application:
**16.08.89 Bulletin 89/33**

(84) Designated Contracting States:
**AT BE CH DE ES FR GR IT LI LU NL SE**

(71) Applicant: **CRAMMOND INTERNATIONAL LIMITED**
**Acorn House Victoria Road**
**London W3 6UL (GB)**

**Lewis, Philip**
**The Garden Flat 13, Warrington Crescent**
**London W9 1ED (GB)**

(72) Inventor: **Kerner, Edward Phillip**
**77 West Heat Road**
**Hampstead London NW3 7TH (GB)**

**Lewis, Philip**
**The Garden Flat No. 13, Warrington Crescent**
**London W9 1ED (GB)**

(74) Representative: **Hackett, Sean James et al**
**Marks & Clerk 57-60 Lincoln's Inn Fields**
**London WC2A 3LS (GB)**

(54) **A method of releasing a fragrance and an apparatus therefor.**

(57) An apparatus for releasing fragrance into an environment is described. The apparatus comprises the combination of a holder (1) for holding a fragrance medium and, as the fragrance medium, a particulate plastics material having a fragrance incorporated therein. The holder (1) is configured to be located on or adjacent to an electric light bulb. The heat from the electric light bulb causes the particulate plastics material to release the fragrance into the environment.

EP 0 328 370 A2

Description

## A METHOD OF RELEASING A FRAGRANCE AND AN APPARATUS THEREFORE

This invention relates to a method of, and to apparatus for, dispensing or releasing fragrance into an environment such as, for example, a room.

It is known, on the one hand, to employ for this purpose an annular trough for location upon an electric light bulb and to use as the fragrance medium a scented oil. When the electric light bulb is switched on, the heat generated by the filament heats the oil and causes the fragrance to be released. It is also known to use for the same purpose a small tray which clips onto a light bulb and which is used with scented wax granules, the device working in the same way as the aforementioned annular trough and oil.

On the other hand, there is known a plastics moulding material which has chemically or physically incorporated therein a fragrance. The material is used to mould articles which are then inherently fragrant.

According to a first aspect of the present invention, there is provided a method of releasing a fragrnce into an environment which comprises locating adjacent an artificial heat source, e.g. an electric light bulb, a holder for a fragrance medium which is to be heated by the heat source, and employing as the fragrance medium to be heated a particulate plastics material incorporating a fragrance.

According to a second aspect of the present invention there is provided apparatus for releasing fragrance into an environment, which apparatus comprises the combination of a holder for holding a fragrance medium and, as the fragrance medium, a particulate plastics material having a fragrance incorporated therein, said holder being adapted to be located adjacent an artificial heat source, e.g. an electric light bulb.

Preferably the fragrance medium comprises granules or beads of polymeric plastics incorporating a fragrance. Preferably, the medium is 'Polyiff' ('Polyiff' is a Trade Mark) available from International Flavors and Fragrances Incorporated, of Hazlet, New Jersey, 07730, United States of America. The granules or beads may be solid or may be in 'expanded' form. Any suitable plastics material may be used as the vehicle for the fragrance; examples of suitable plastics are polyethylene, polypropylene and polyvinyl chloride.

The holder may be in the form of a generally annular trough for location about an electric light bulb or may be in the form of a tray or container supported by one or more spring clips for mounting upon an electric light bulb. The holder may be made of any suitable heat-resistant material such as for example, a metal, a thermosetting resin, or a ceramic material such as porcelain or earthenware.

It has however been found, surprisingly, that some thermoplastic resins are capable of resisting the heat generated by an electric light bulb; in a preferred embodiment, therefore, the holder is in the form of a trough to be supported by an electric light bulb and is made from nylon 6,6 or preferably celanex 2002.

An embodiment of apparatus according to the present invention and of the method thereof, will now be described by way of example only, with reference to the accompanying drawing, in which drawing:

Fig. 1 is a plan view of an embodiment of a holder according to the present invention;

Fig. 2 is a cross-section along the line B-B of the holder of Fig. 1;

Fig. 3a is a plan view of another embodiment of the present invention;

Fig. 3b is an underneath view of the embodiment of Fig. 3a;

Fig. 3c is a side elevational view of the embodiment of Fig. 3a; and

Figs. 3d and 3e are sectional views of the embodiment of Fig. 3a.

Referring to the drawing, there is shown a holder 1 for a fragrance medium in the form of an annular trough 2 of semi-circular cross-section, the central aperture 3 defined by the annular trough 2 having a diameter of 56 mm. The trough 2 has a maximum depth of 10 mm. The holder 1 is provided about its outer edge with decorative, approximately semi-circular lobes or petals 4 and has a minimum diameter of 90 mm. The holder 1 may be made of white nylon 6,6, but is preferably made of celanex 2002 which is a form of plastics material. The holder 1 is adapted to seat upon a 40 watt electric light bulb of conventional pattern and is heat-resistant to approximately 200° C.

The holder 1 is adapted to receive a particulate fragrance medium in the form of 'polyiff' beads or granules. Two grades of these granules are available and either may be used. The standard 'polyiff' beads are of low density polyethylene and have the following properties:

| | | |
|---|---|---|
| Melt Index | : | 20 |
| Density | : | 0.9-0.93 |
| Bulk Density | : | 28-37 lb/ft$^3$ |
| Pellet Size (cylindrical) | : | 0.12x0.185" nominal |
| Colour | : | Neutral-light brown. |

Puffed or expanded 'polyiff' beads are preferred for use in the present invention. They too are of low density polyethylene and have the following properties:

| | | |
|---|---|---|
| Bulk Density | : | 15-27 lb/ft$^3$ |
| Shape/size | : | approx 1/8" spheres |
| Colour | : | 12 decorative colours available |

As has been previously indicated the particulate 'polyiff' material can be made from polypropylene, polyvinyl chloride or another suitable polymer.

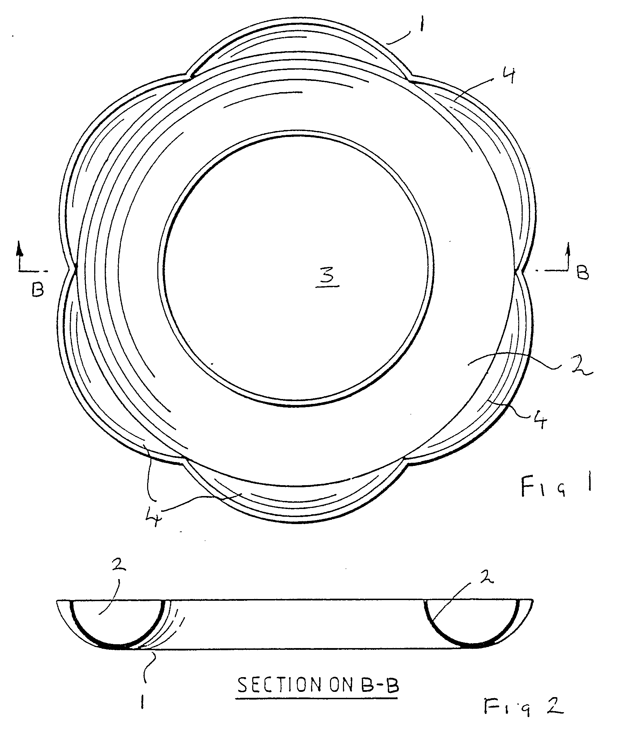

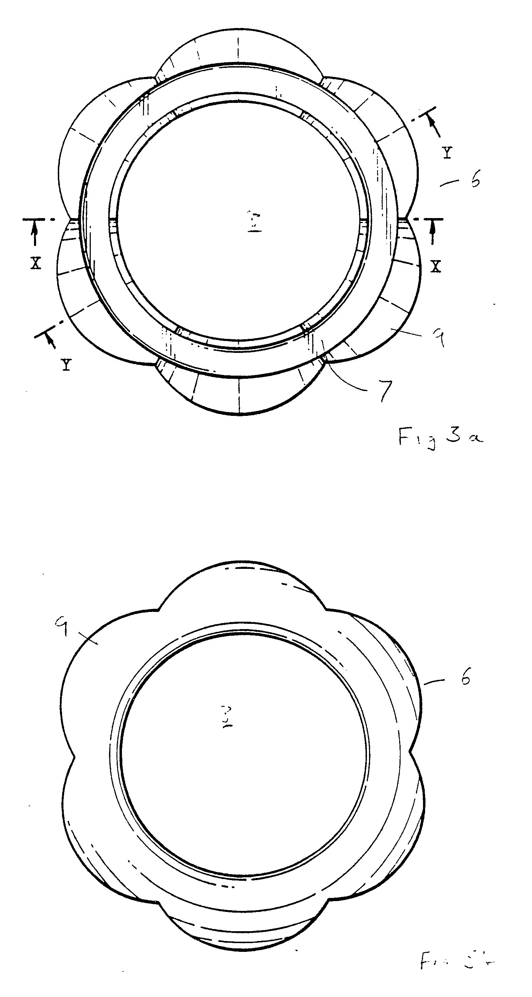

To use the holder and fragrance medium described with reference to Figs 1 and 2, the holder may be seated on a 40 watt electric light bulb and the trough at least partially filled with puffed 'polyiff' beads. When electricity is supplied to the light bulb, the 'polyiff' beads are heated and so release their fragrance into the environment.

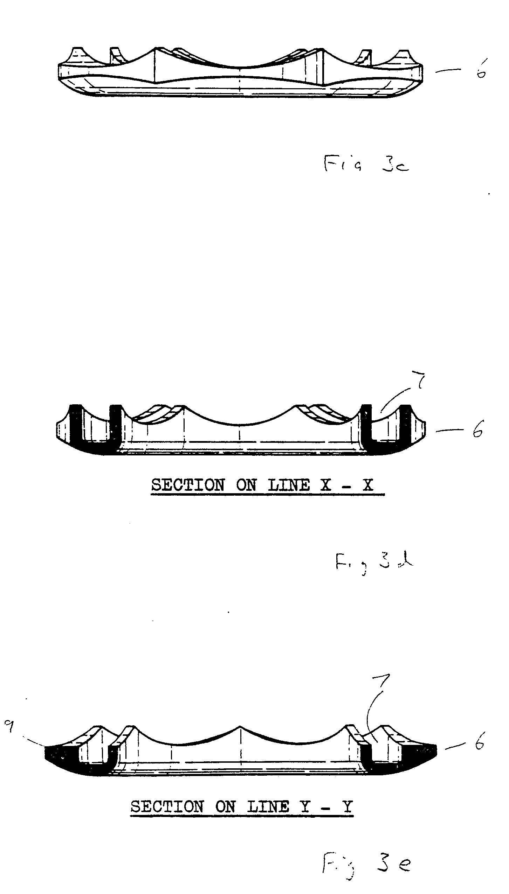

Figures 3a to 3e show an alternative form of holder 6. The holder 6 has an annular trough 7 for receiving and holding the granules. The holder 6 has a central aperture 8 for enabling the holder 6 to sit upon an electric light bulb. The outer edge of the holder is provided with approximately semi-circular lobes or petals 9.

The method and apparatus of the present invention provide a novel and useful way of releasing a fragrance into an environment, using existing materials in a novel and surprising way.

## Claims

1. A method of releasing a fragrance into an environment, characterised by locating adjacent an artificial heat source, a holder (1, 6) for 1a fragrance medium which is to be heated by the heat source, and employing as the fragrance medium to be heated a particulate plastics material incorporating the fragrance.

2. A method according to Claim 1, wherein the artificial heat source is an electric light bulb.

3. A method according to Claim 1 or 2, wherein the particulate plastics material comprises granules or beads of polymeric plastics incorporating a fragrance.

4. Apparatus for releasing fragrance into an environment, characterised in that the apparatus comprises the combination of a holder (1, 6) for holding a fragrance medium and, as the fragrance medium, a particulate plastics material having a fragrance incorporated therein, said holder being adapted to be located adjacent an artificial heat source.

5. The combination of Claim 4, wherein the fragrance medium comprises a polymeric plastics material incorporating a fragrance.

6. A method according to claim 3 or the combination according to claim 5, wherein the polymeric plastics material is selected from the group consisting of polyethylene, polypropylene and polyvinyl chloride.

7. A method according to any of claims 1 to 3, or the combination according to any of claims 4, 5 or 6, wherein the holder (1, 6) is in the form of a generally annular trough (2, 7) adapted for location about an electric light bulb.

8. A method according to any one of claims 1 to 3, or the combination according to any of claims 4 to 7, wherein the holder is selected from one of nylon 6, 6, celanex 2002 or metal.

9. A method according to any of claims 1 to 3, or the combination according to any of claims 4 to 8, wherein the holder is in the form of a container supported by spring clip means adapted for mounting the holder upon an electric light bulb.

Fig 1

SECTION ON B-B

Fig 2

Fig 3a

Fig 3b

Fig 3c

SECTION ON LINE X - X

Fig 3d

SECTION ON LINE Y - Y

Fig 3e